# FASCICULE DE BREVET EUROPEEN

(11) **EP 4 041 336 B1**
(45) Date de publication et mention de la délivrance du brevet: **02.07.2025**
(21) Numéro de dépôt: 20789599.6
(22) Date de dépôt: 12.10.2020
(51) Int. Cl.: C12M 1/00

(54) **SYSTEME POUR LA LIBERATION DE PLAQUETTES ET PROCEDE DE LIBERATION DE PLAQUETTES**
SYSTEM ZUR FREIGABE VON BLUTPLÄTTCHEN UND VERFAHREN ZUR FREIGABE VON BLUTPLÄTTCHEN
SYSTEM FOR THE RELEASE OF PLATELETS AND METHOD FOR RELEASING PLATELETS

(30) Priorité: 11.10.2019 FR 1911303
(43) Date de publication de la demande: 17.08.2022
(73) Titulaire: Etablissement Français du Sang, 93210 La Plaine Saint Denis (FR); Avignon Universite, 84029 Avignon Cedex 1 (FR); Institut National de la Santé et de la Recherche Médicale, 75013 Paris (FR); Université de Strasbourg, 67081 Strasbourg Cedex (FR)
(72) Inventeur: DO SACRAMENTO, Valentin, 67280 Urmatt (FR); KNAPP, Yannick, 13013 Marseille (FR); MALLO, Léa, 67100 Strasbourg (FR); STRASSEL, Catherine, 67000 Strasbourg (FR)
(74) Mandataire: Gevers & Orès
(86) Numéro de dépôt international: PCT/EP2020/078639
(87) Numéro de publication internationale: WO 2021/069747

(56) Documents cités:
- WO-A1-2010/063823
- WO-A1-94/08692
- WO-A2-2009/131645
- US-A- 6 008 040
- YUKITAKA ITO ET AL: "Turbulence Activates Platelet Biogenesis to Enable Clinical Scale Ex Vivo Production", CELL, vol. 174, no. 3, 1 July 2018 (2018-07-01), AMSTERDAM, NL, pages 636 - 648.e18, XP055567213, ISSN: 0092-8674, DOI: 10.1016/j.cell.2018.06.011
- ALAINA C. SCHLINKER ET AL: "Separation of in-vitro-derived megakaryocytes and platelets using spinning-membrane filtration : Separation of In-Vitro-Derived Platelets", BIOTECHNOLOGY AND BIOENGINEERING, vol. 112, no. 4, 19 November 2014 (2014-11-19), US, pages 788 - 800, XP055376178, ISSN: 0006-3592, DOI: 10.1002/bit.25477

## Description

### Domaine technique de l'invention

L'invention concerne un système pour la libération de plaquettes à partir d'un fluide comprenant notamment des progéniteurs de mégacaryocytes, cellules mégacaryocytaires comportant des extensions cytoplasmiques. L'invention concerne également un ensemble muni d'une pluralité de systèmes tel que celui-ci. Enfin, l'invention concerne un procédé de libération continue de plaquettes au moyen d'un tel système. L'invention est plus particulière adaptée à la production in vitro de plaquettes sanguines à une échelle industrielle.

### Arrière-plan technique

La production in vitro de plaquettes sanguines répond à des besoins croissants dans diverses applications médicales. Actuellement, il existe essentiellement deux catégories de systèmes destinés à cet usage spécifique: les systèmes microfluidiques et les systèmes « à réservoir ». Ces catégories de systèmes destinés à libérer les plaquettes sont issues d'observations réalisées in situ qui montrent le besoin d'un flux.

Un exemple de système à réservoir est décrit dans le document WO201909364 A1. Le système est muni de moyens d'agitation du fluide à traiter. Il comprend un réservoir pour le fluide comportant notamment les cellules mégacaryocytaires et au moins un moyen pour agiter le fluide, l'agitation étant à l'origine de la libération des plaquettes. Cette agitation est générée à l'intérieur du réservoir lui-même, par exemple, au moyen d'une pâle en va et vient vertical. L'inconvénient principal d'un tel système est qu'il n'est pas apte à fonctionner dans des conditions garantissant la qualité des plaquettes ainsi obtenues puisqu'il n'est pas isolé de l'environnement extérieur et des sources de contamination présentes dans cet environnement, ce qui le rend impropre à une utilisation WO2009131645 divulgue un appareil de ségrégation de cellules en fonction de leur capacité à s'écouler dans un passage à gradins. médicale.

Les systèmes microfluidiques comme leur nom l'indique sont généralement des systèmes de dimensions micrométriques (quelques dizaines de microns à quelques centaines de microns tout au plus) comprenant un réseau de réservoirs et de canaux interconnectés selon un agencement particulier en vue de remplir différentes fonctions. Ils comprennent, de manière classique, un site dédié à la culture de cellules mégacaryocytaires ou mégacaryocytes, et éventuellement à la libération des plaquettes, le site étant relié à un réseau de canaux configurés pour extraire/récupérer les plaquettes. Ces systèmes, pour la plupart biomimétiques, cherchent à reproduire l'environnement physiologique afin d'accroître la production de plaquettes.

Du fait de leur petite taille, ces systèmes microfluidiques présentent deux limites intrinsèques. La première étant le faible débit de plaquettes pouvant être atteint par de tels systèmes, généralement de quelques centaines de microlitres par heure (µL/h). À titre d'exemple, un système ayant un débit de 200 µL/h nécessiterai 50 000 heures, soit 5,7 ans pour traiter 10 L de fluide. Il est alors nécessaire de connecter plusieurs systèmes en parallèle pour obtenir un débit total comparable. Cela étant, dans l'exemple ci-dessus le nombre de systèmes requis serait très élevé, i.e. 50 000, et la complexité du système serait encore plus accrue. Au mieux de tels systèmes ne peuvent donc convenir que pour des volumes limités d'échantillons de fluide.

En outre, les dispositifs microfluidiques décrits dans la littérature mettent tous en œuvre un mécanisme/procédé qui "fixe" les mégacaryocytes puis en extrait les plaquettes. Cette phase de fixation est souvent basée sur l'utilisation d'une drogue ou d'un composé chimique de revêtement, ce qui constitue un inconvénient.

Plus récemment, un nouveau procédé de libération des plaquettes à partir de cellules mégacaryocytaires en ayant recours au pipetage a été développé. Un tel procédé est décrit dans le document Strassel et al. « Aryl hydrocarbon receptor-dependent enrichment of a high potential to produce propalets », Blood, 5 Mai 2016, vol. 127, n° 18. Dans un tel procédé, le processus de libération des plaquettes résulte directement du pipetage. Le pipetage dont il s'agit est similaire à un pipetage classique puisqu'il consiste à prélever un échantillon d'un milieu comprenant des cellules mégacaryocytaires au moyen d'une pipette à ceci près que l'action de pipeter est répétée autant de fois que nécessaire afin de créer l'agitation nécessaire à la libération des plaquettes. À l'issu de ce procédé, des plaquettes ont été détectées dans le fluide traité, validant ainsi la capacité du pipetage pour la libération des plaquettes, qui plus est avec une grande simplicité de mise en œuvre. Cependant, ce procédé n'est pas adapté à une exploitation à grande échelle puisque le pipetage est par nature manuel et qu'en conséquent il n'a vocation qu'à traiter des volumes limités de fluide.

### Résumé de l'invention

L'invention permet de surmonter les inconvénients précités et propose à cet effet un système de libération des plaquettes à partir d'un fluide comprenant notamment des cellules mégacaryocytaires comportant des extensions cytoplasmiques, ledit système comprenant :
- un dispositif comprenant :
   ∘ un réservoir de libération de plaquettes comprenant une première ouverture et une deuxième ouverture,
   ∘ un premier élément de raccordement fluidique fixé au niveau de ladite première ouverture et adapté pour injecter ledit fluide à l'intérieur dudit réservoir, ledit premier élément de raccordement comprenant un orifice d'injection du fluide dans le réservoir de libération de plaquettes et une portion étrécie en direction dudit orifice, un élargissement brusque de section existant entre l'orifice d'injection et le réservoir,
   ∘ un deuxième élément de raccordement fluidique fixé au niveau de ladite deuxième ouverture, ledit deuxième élément de raccordement comprenant un orifice de refoulement du fluide,
- un dispositif de pompage du fluide en communication fluidique avec le réservoir par l'intermédiaire du deuxième élément de raccordement fluidique ou du premier élément de raccordement fluidique,
- un module d'alimentation électrique du dispositif de pompage,
- un système de programmation configuré pour contrôler le module d'alimentation électrique du dispositif de pompage, afin de mettre en œuvre une ou plusieurs séquences de libération de plaquettes réalisées de sorte à générer un écoulement continu du fluide entre ledit orifice d'injection et ledit orifice de refoulement ainsi que des perturbations tourbillonnaires au sein du réservoir provoquant la fragmentation des extensions cytoplasmiques des cellules mégacaryocytaires.

Le système selon l'invention est ainsi configuré de manière à reproduire le processus de pipetage effectué manuellement au moyen d'une pipette ce qui autorise une libération continue et automatique des plaquettes.

Ceci s'effectue toutefois à grande échelle car la géométrie du système selon l'invention permet de traiter des volumes importants de fluide avec un débit élevé (de l'ordre de plusieurs litres par heure), ce qui le rend particulièrement adapté à une utilisation à l'échelle industrielle.

En outre, le système permet d'obtenir un nombre de plaquettes libérées à partir des cellules mégacaryocytaires au moins équivalent à celui obtenu à partir du pipetage manuel, ce qui en fait un système présentant un rendement de libération de plaquettes particulièrement élevé. Ceci est obtenu principalement du fait de la synergie entre la forme des éléments du système, i.e. notamment la conicité du premier élément de raccordement fluidique et les dimensions importantes du réservoir, et la vitesse d'écoulement générée par le dispositif de pompage. Cela permet de générer des perturbations tourbillonnaires sensiblement de la taille des mégacaryocytes au sein du réservoir.

Selon différentes caractéristiques de l'invention qui pourront être prises ensemble ou séparément :
- la portion étrécie est conique ;
- le premier élément de raccordement comprend un axe longitudinal et le deuxième élément de raccordement comprend un axe longitudinal, lesdits axes longitudinaux étant soit sécants soit parallèles et alors séparés d'une distance, d, non nulle ;
- l'orifice d'injection présente un diamètre d'ouverture inférieur à 1 mm ;
- un rapport du diamètre d'ouverture de l'orifice d'injection par une largeur de section du réservoir est compris entre 0,02 et 0,1 ;
- le rapport du diamètre d'ouverture de l'orifice d'injection par la largeur de section du réservoir est de 0,05 ;
- l'orifice de refoulement présente un diamètre d'ouverture inférieur à 1 mm, un rapport du diamètre d'ouverture de l'orifice de refoulement par une largeur de section du réservoir est compris entre 0,02 et 0,1 ;
- le rapport du diamètre d'ouverture de l'orifice de refoulement par la largeur de section du réservoir est de 0,05 ;
- le réservoir présente une forme sphérique ;
- le système comprend un réservoir source pour le stockage du fluide, connecté au premier élément de raccordement fluidique pour alimenter le réservoir de libération des plaquettes ;
- le système comprend un réservoir de réception du fluide connecté au deuxième élément de raccordement fluidique pour collecter ledit fluide destiné à être aspiré du réservoir de libération des plaquettes ;
- le dispositif de pompage est situé dans le réservoir de réception ;
- ledit deuxième élément de raccordement comprend en outre une portion évasée depuis ledit orifice de refoulement du fluide en direction du dispositif de pompage ;
- le système comprend un autre dispositif, ledit autre dispositif comporte :
   ∘ un réservoir de libération de plaquettes comprenant une première ouverture et une deuxième ouverture,
   ∘ un premier élément de raccordement fluidique fixé au niveau de ladite première ouverture et adapté pour injecter ledit fluide à l'intérieur dudit réservoir, ledit premier élément de raccordement comprenant un orifice d'injection du fluide et une première portion étrécie de sorte à pouvoir accélérer le fluide, ladite première portion étrécie débouchant sur ledit orifice d'injection,
   ∘ un deuxième élément de raccordement fluidique fixé au niveau de ladite deuxième ouverture, ledit deuxième élément de raccordement comprenant un orifice de refoulement du fluide,
   ledit autre dispositif étant agencé parallèlement au premier dispositif et connecté au dispositif de pompage par l'intermédiaire du deuxième dudit un autre dispositif ;
- le système comprend un autre dispositif, l'autre dispositif comporte :
   ∘ un réservoir de libération de plaquettes comprenant une première ouverture et une deuxième ouverture,
   ∘ un premier élément de raccordement fluidique fixé au niveau de ladite première ouverture et adapté pour injecter ledit fluide à l'intérieur dudit réservoir, ledit premier élément de raccordement comprenant un orifice d'injection du fluide et une première portion étrécie de sorte à pouvoir accélérer le fluide, ladite première portion étrécie débouchant sur ledit orifice d'injection,
   ∘ un deuxième élément de raccordement fluidique fixé au niveau de ladite deuxième ouverture, ledit deuxième élément de raccordement comprenant un orifice de refoulement du fluide,
   ledit autre dispositif étant agencé en série avec le premier dispositif, ledit deuxième élément de raccordement dudit autre dispositif étant en communication fluidique avec ledit premier élément de raccordement fluidique dudit premier dispositif.

L'invention concerne en outre un procédé de libération de plaquettes à partir d'un fluide comprenant notamment des cellules mégacaryocytaires comprenant des extensions cytoplasmiques, ledit procédé comprenant les étapes suivantes, mises en œuvre au moyen d'un système tel que précédemment décrit :
(100) fournir un fluide comprenant des cellules mégacaryocytaires en suspension dans ledit fluide, lesdites cellules mégacaryocytaires comprenant des extensions cytoplasmiques,
(200) alimenter électriquement le dispositif de pompage,
(300) commander le système de programmation afin de lancer une ou plusieurs séquences de libération de plaquettes
la ou chaque séquence de libération de plaquettes étant réalisée de sorte à générer un écoulement continu du fluide entre ledit orifice d'injection et ledit orifice de refoulement ainsi que des perturbations tourbillonnaires au sein du réservoir provoquant la fragmentation des extensions cytoplasmiques des cellules mégacaryocytaires.

Avantageusement, lors de l'étape (200), un vide relatif compris entre -10 kPa et -50 kPa est généré dans le dispositif.

### Brève description des figures

D'autres objets et caractéristiques de l'invention apparaîtront plus clairement dans la description qui suit, faite en référence aux figures annexées, dans lesquelles :
[Fig. 1a] La figue 1a est une représentation schématique d'un système selon un premier mode de réalisation de l'invention, un réservoir pour le fluide étant illustré en section selon une vue de côté ;
[Fig. 1b] La figue 1b est une représentation schématique d'une variante de réalisation du système de la figure 1a;
[Fig. 1c] La figure 1c est une vue rapprochée d'un réservoir équipant le système illustré à la figure 1b ;
[Fig. 2a] La figure 2a est une représentation schématique d'un système selon un deuxième mode de réalisation de l'invention comprenant une pluralité de dispositifs montés en série ;
[Fig. 2b] La figure 2b représente une vue éclatée d'un système selon l'invention comprenant cinq dispositifs montés en série ;
[Fig. 2c] La figure 2c représente un ensemble tel qu'illustré aux figures 2a et 2b, l'ensemble est ici connecté à un réservoir source et un réservoir de réception du fluide ;
[Fig. 2d] La figure 2d est une représentation schématique d'un ensemble selon un troisième mode de réalisation de l'invention, comprenant une pluralité de dispositifs montés en parallèle ;
[Fig. 3a] La figure 3a est une représentation schématique d'un procédé selon l'invention ;
[Fig. 3b] La figure 3b est une représentation schématique du procédé de la figure 3a dans laquelle sont illustrées les sous-étapes de réalisation du procédé ;
[Fig. 4a] La figure 4a illustre une cellule mégacaryocytaire proplaquettaire ;
[Fig. 4b] La figure 4b illustre des plaquettes obtenues après le procédé de libération de plaquettes selon l'invention ;
[Fig. 5a] La figure 5a est une figure comparative illustrant le rendement de libération de plaquettes obtenu en utilisant une pipette et en utilisant un système selon l'invention en fonction du vide relatif au sein dudit système ;
[Fig. 5b] La figure 5b est une analyse de la fonctionnalité des plaquettes obtenues à une pression de -30 kPa pour des plaquettes natives (barres creuses) et des plaquettes de culture (barres pleines).

### Description détaillée de l'invention

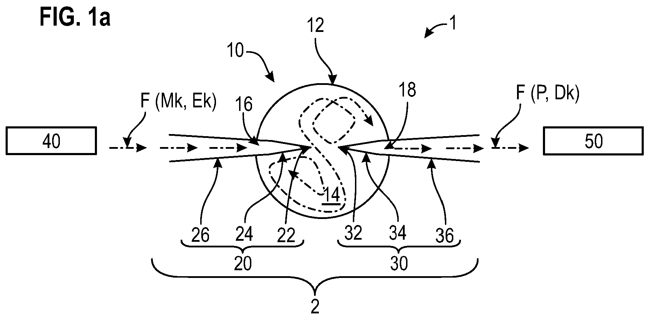

En référence aux figures 1a à 1c, l'invention concerne un système 1 pour la libération de plaquettes P à partir d'un fluide F comprenant des cellules mégacaryocytaires Mk.

Le fluide F dont il s'agit est par exemple un milieu de culture contenant une population de cellules obtenues à partir de cellules souches immortalisées ou non à différents stades de différenciation dont des progéniteurs de mégacaryocytes, des mégacaryocytes. Les mégacaryocytes ou cellules mégacaryocytaires sont des cellules sanguines de grande taille (jusqu'à 100 µm et 30 µm en culture) qui lorsqu'elles ont atteint la maturité présentent de longues extensions appelées extensions cytoplasmiques ou prolongements cytoplasmiques ou encore proplaquettes.

Les mécanismes intervenant dans la formation des plaquettes sanguines font encore l'objet de nombreuses recherches. Parmi eux, la libération de plaquettes intervient au cours d'un processus de fragmentation des mégacaryocytes Mk et/ou des extensions cytoplasmiques, Ck, en plaquettes. Il s'agit d'un processus *in vivo* très coordonné se produisant de manière naturelle dans le sang grâce à la force du flux sanguin. Les mégacaryocytes Mk y jouent un rôle essentiel puisque ce sont des cellules précurseurs. Cela étant, le processus de libération des plaquettes dans l'organisme reste peu décrit et le passage transendothélial et le rôle précis du flux sanguin dans la formation des plaquettes soulèvent encore de nombreuses interrogations. Ce processus a été reproduit in vitro au moyen de systèmes microfluidiques ce qui a permis de corroborer certains mécanismes *in vivo* par des expériences microfluidiques. Ce processus peut également être reproduit *in vitro* au moyen d'une pipette manuellement ou au moyen de dispositifs tels que ceux présentés en partie introductive de cette description, ce qui permet également de mieux comprendre les mécanismes impliqués dans la libération des plaquettes (Strassel *et al.* 2016). La production de plaquettes in vitro permet de mieux comprendre les mécanismes intervenant dans la formation des plaquettes. Cependant, de manière générale les rendements de libération des plaquettes sont moindres que ceux obtenus *in vivo.* Le dispositif proposé par l'invention permet de mimer le processus de libération de plaquettes par pipetage manuel divulgué par Strassel *et al.,* sans pour autant reproduire le mouvement de va-et-vient généré lors du pipetage qui est remplacé par un mouvement continu en utilisant le système 1. L'invention vise ainsi à reproduire le processus de fragmentation des mégacaryocytes Mk et/ou des extensions cytoplasmiques Ck à une échelle industrielle et permet donc de traiter de grands volumes de fluide F (plusieurs litres par heure).

Le système 1 selon l'invention comprend un dispositif 2, un dispositif de pompage 60 et un système de programmation 70 que nous allons décrire dans les sections qui suivent.

Le dispositif 2 comprend un réservoir 10 de libération de plaquettes, un premier élément 20 de raccordement fluidique et un deuxième élément 30 de raccordement fluidique.

Le réservoir 10 de libération de plaquette (ci-après nommé « réservoir 10 ») présente des dimensions longitudinales millimétrique à centimétrique, c'est-à-dire des dimensions longitudinales comprises entre 1 mm et quelques centimètres. À titre de comparaison, la dimension la plus petite du réservoir 10 du système 1 selon l'invention est la dimension la plus grande d'un système microfluidique traditionnel. Ceci en fait donc un réservoir de dimensions et de capacité plus importantes que ceux des systèmes microfluidiques connus.

Dans l'exemple de réalisation illustré à la figure 1a, le réservoir 10 est de forme sphérique. Il comporte une paroi 12 de forme sphérique délimitant une cavité 14 au sein de laquelle le fluide F peut circuler. En plus d'être une zone pour la circulation pour le fluide F, la cavité est une zone permettant un écoulement turbulent du fluide F, c'est-à-dire permettant la formation de perturbations tourbillonnaires de tailles au moins égales à celles des mégacaryocytes Mk. En d'autres termes, la cavité 14 par ses dimensions, telles que précédemment décrites, d'une part et par son volume intérieur d'autre part est configurée de sorte à permettre la formation de perturbations tourbillonnaires en son sein. Toutefois, le réservoir 10 pourrait être de toute autre forme, par exemple, parallélépipédique, cylindrique, etc., le plus important étant que ledit réservoir 10 comprend une cavité 14 de dimensions suffisantes pour permettre la formation des perturbations tourbillonnaires. Par exemple, pour un fluide F comprenant des mégacaryocytes Mk dont, on l'a vu, la taille peut atteindre 30 µm en culture, la cavité 14 présente donc des dimensions nettement supérieures (millimétrique à centimétrique) puisque le fluide F comprend une pluralité de cellules mégacaryocytaires Mk, par exemple des centaines voire des milliers.

La paroi 12 comprend au moins une première ouverture 16 et une deuxième ouverture 18. La première ouverture 16 est dédiée au raccordement fluidique avec le premier élément 20 de raccordement, tandis que la deuxième ouverture 18 est dédiée au raccordement fluidique avec le deuxième élément 30 de raccordement. De préférence, elles forment les seules ouvertures de la paroi 12 puisque, le système 1 est fermé.

Le premier élément 20 de raccordement fluidique est un moyen d'amenée du fluide F au sein du réservoir 10. Autrement dit, il s'agit d'une entrée pour le fluide F au sein du réservoir 10. Il est fixé au niveau de la première ouverture 16 du réservoir 10. Il comprend une portion 26 de liaison, une portion 24 étrécie et un orifice 22 d'injection du fluide dans cet ordre dans le sens d'écoulement du fluide. La portion 24 étrécie débouche donc sur l'orifice 22 d'injection. Ceci étant dit, on notera que la portion 26 de liaison n'est pas indispensable comme ceci sera mieux compris dans la suite. Ainsi, dans l'exemple de réalisation illustré à la figure 1a, le premier élément 20 de raccordement fluidique est fixé au niveau de la première ouverture 16 par l'intermédiaire de la portion 26 de liaison, mais il peut être fixé au réservoir 10 selon d'autres configurations. En effet, il peut tout aussi bien être fixé par l'intermédiaire de la portion 24 étrécie comme par l'intermédiaire de l'orifice 22 d'injection. Dans cette dernière configuration, on comprendra alors que l'orifice 22 d'injection, en étant localisé au niveau de la paroi 12, peut correspondre à, i.e. confondu avec, la première ouverture 16 du réservoir.

Comme mentionné précédemment, la portion 24 est étrécie. La portion 24 est étrécie en ce qu'elle se rétrécit depuis la portion 26 de liaison ou, alternativement en l'absence de portion 26 de liaison, une extrémité du premier élément 20 de raccordement en direction de l'orifice 22 d'injection du fluide. Autrement dit, elle se rétrécit dans le sens de l'écoulement relativement à un axe longitudinal X1 dudit premier élément 20 de raccordement passant par le centre dudit orifice 22. La forme étrécie de ladite portion 24 permet d'accélérer le fluide F par effet venturi. Incidemment, le fluide F entrant dans le réservoir 10 présente donc une vitesse plus importante comparativement à un fluide qui pénétrerait dans le système 1 à travers une portion 24 qui ne présente pas d'évasement.

De manière avantageuse, le rétrécissement de la portion 24 étrécie depuis la portion 26 de liaison ou, alternativement en l'absence de portion 26 de liaison, l'extrémité du premier élément 20 de raccordement en direction de l'orifice 22 d'injection du fluide peut être sensiblement constant. La constance du rétrécissement permet de réduire les frottements et d'augmenter encore plus l'accélération du fluide. À cet égard, la portion 24 étrécie peut être conique. Avantageusement, l'utilisation d'une portion 24 conique facilite la connexion avec les moyens de connexion fluidiques tubulaires, i.e. de forme cylindrique, fréquemment vendus dans le commerce. À titre d'exemples, la portion 24 peut présenter une forme pyramidale, tétraédrique, etc. Ce qui importe avant tout est que la portion 24 étrécie se rétrécisse depuis la portion 26 de liaison du premier élément 20 de raccordement en direction de l'orifice 22 d'injection du fluide. Cela étant, une fois refoulé dans le réservoir 10, le fluide F ralentit substantiellement du fait de la différence de section existant entre le premier élément 20 de raccordement fluidique, notamment l'orifice 22 d'injection, et le réservoir 10.

En effet, l'orifice 22 d'injection présente avantageusement un diamètre d'ouverture de très faible dimension par rapport au diamètre du réservoir 10, dans le cas d'espèce sphérique. Toutefois, ce diamètre est supérieur ou égal à la taille des particules sortant du réservoir 10, i.e. les plaquettes P et d'autres produits, Dk, résultants essentiellement de la fragmentation des mégacaryocytes. De préférence, le diamètre d'ouverture est inférieur ou égal à 1 mm, alors que nous l'avons vu le réservoir 10 présente des dimensions longitudinales millimétrique à centimétrique, en tout état de cause bien supérieures à celles de l'orifice 22. Précisons à ce stade que la forme de l'orifice 22 d'injection est non limitative. Ce qui importe ici est la taille dudit orifice 22 d'injection.

Ainsi, un élargissement brusque de section existe entre l'orifice 22 d'injection et le réservoir 10. Cet élargissement brusque correspond à une singularité et donne lieu à un profil d'écoulement singulier du fluide F. Comme mentionné précédemment, le fluide F est accéléré en sortie de la portion 24 étrécie à travers l'orifice 22 d'injection, puis subit une perte de charge, i.e. un ralentissement, lors de son passage de l'orifice 22 d'injection au réservoir, du fait de cette singularité. Plus le rapport, R_{E}, du diamètre de l'orifice 22 d'injection par le diamètre ou, plus généralement, la largeur en section du réservoir 10 est petit devant 1 plus la singularité sera accentuée plus la perte de charge est importante et inversement lorsque ce rapport augmente. Un exemple de système 1 selon l'invention ayant été mis en œuvre comprend un réservoir 10 de diamètre égal à 16 mm et un orifice 22 d'injection de diamètre égale à 0,8 mm (R_{E} est donc égal à 0,05). Si un tel système 1 permet d'obtenir des rendements de libérations de plaquettes améliorés, des rapports R_{E} du diamètre d'ouverture de l'orifice 22 par la largeur de section du réservoir compris entre 0,02 et 0,1 peuvent aussi être envisagés pour libérer des plaquettes à partir d'un fluide F, toujours pour un orifice 22 d'injection de diamètre d'ouverture inférieur à 1 mm. Avantageusement, R_{E} est compris entre 0,04 et 0,08 un diamètre d'ouverture inférieur à 1 mm. Plus avantageusement, R_{E} est compris entre 0,04 et 0,06 un diamètre d'ouverture inférieur à 1 mm. Cela étant dit, encore plus avantageusement R_{E} est égal à 0,05. En effet, dans ce dernier cas, le rendement de libération des plaquettes est meilleur.

Pour autant, comme cela sera explicité plus loin, bien que le fluide F est ralenti en entrant dans le réservoir 10 et conserve un régime d'écoulement laminaire au sens stricte du terme - le nombre de Reynold variant entre 0, au centre d'un tourbillon dont la taille est proche de celle du réservoir 10, et 1500, au niveau de l'orifice 22 d'injection, il n'est pas immobile au sein dudit réservoir 10 du fait du déplacement de fluide généré par le dispositif de pompage 60. Le réservoir 10 n'est donc qu'un lieu de passage pour le fluide F, et n'est pas un lieu de stockage dudit fluide F, c'est-à-dire un lieu dans lequel le fluide est amené à stagner lorsque le système 1 est en fonctionnement.

À ce propos, précisons qu'en plus du réservoir 10 de libération des plaquettes, le système 1 peut également comprendre un réservoir 40 source pour le stockage du fluide F connecté au premier élément 20 de raccordement fluidique pour alimenter le réservoir. Le fluide F peut donc être stocké dans le réservoir 40 source préalablement à son passage dans le premier élément 20, mais cela n'est pas obligatoire. Le réservoir 40 est donc l'élément le plus en amont du système 1 dans le sens de l'écoulement.

En outre, le système 1 peut également comprendre un réservoir 50 de réception du fluide F. Le réservoir 50 de réception du fluide F est connecté au deuxième élément 30 de raccordement fluidique pour collecter ledit fluide destiné à être aspiré du réservoir. Au contraire du réservoir source 40, le réservoir 50 de réception est l'élément situé le plus en aval du système 1 dans le sens de l'écoulement. L'agencement de ce dernier sera explicité plus loin en relation avec le deuxième élément 30 de raccordement fluidique.

Le réservoir 10 peut donc être vu comme central en ce que le système 1 peut comprendre en amont de celui-ci le réservoir 40 source et en aval de celui-ci le réservoir 50 de réception dans le sens de l'écoulement.

Le deuxième élément 30 de raccordement fluidique est, quant à lui, un moyen d'évacuation du fluide F à l'extérieur du réservoir 10 et forme donc un canal de sortie pour le fluide F. Il est en communication fluidique, via l'une de ses extrémités, avec le réservoir 50 de réception. De plus, il est fixé au niveau de la deuxième ouverture 18 du réservoir. Il comprend un orifice 32 de refoulement du fluide, une portion 34 de refoulement et une portion 36 de liaison dans cet ordre dans le sens d'écoulement du fluide F. Il est agencé de manière similaire au premier élément 10 de raccordement fluidique relativement au réservoir 10, bien qu'étant orienté différemment, et présente une structure similaire (ex : taille de l'orifice 32 de refoulement, vis-à-vis du réservoir 10 de libération de plaquettes).

Cela étant, la géométrie de l'écoulement est différente à l'interface réservoir 10/orifice 32 de refoulement, ce qui s'explique essentiellement par le type de singularité. Au contraire de l'interface orifice 22 d'injection/réservoir 10, la singularité tient à un rétrécissement brusque de section dans le sens de l'écoulement puisque le diamètre (ou pourtour du réservoir 10 le cas échéant) est beaucoup plus important que le diamètre de l'orifice 32 de refoulement. Compte-tenu des dimensions du réservoir 10 et celles de l'orifice 32 de refoulement, une telle singularité aurait pour effet d'augmenter encore plus la perte de charge subie par le fluide F lorsque ce dernier passe du réservoir 10 au deuxième élément 30 de raccordement fluidique, mais ce phénomène est atténué du fait du déplacement de fluide généré par le dispositif de pompage 60, comme cela sera décrit plus en détail dans la suite. Cette singularité permet d'augmenter le temps de résidence du fluide au sein du réservoir 10 puisque le rétrécissement brusque de section agit comme un obstacle pour le fluide F qui est empêché de sortir immédiatement du réservoir 10 sinon directement du réservoir. La conséquence directe de cela est que, le temps de résidence du fluide F étant augmenté, la formation des turbulences ou perturbations tourbillonnaires au sein du réservoir 10 est favorisée.

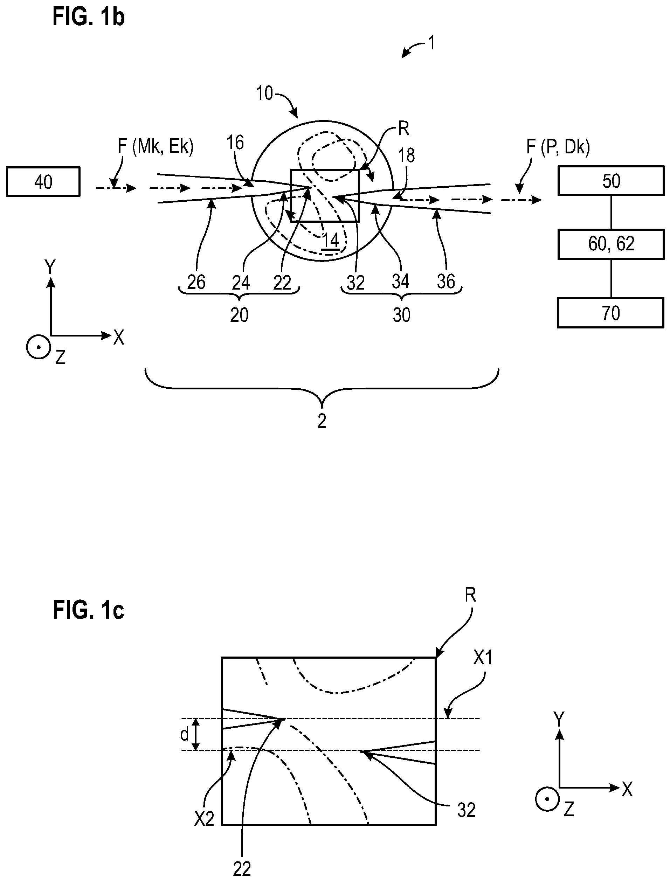

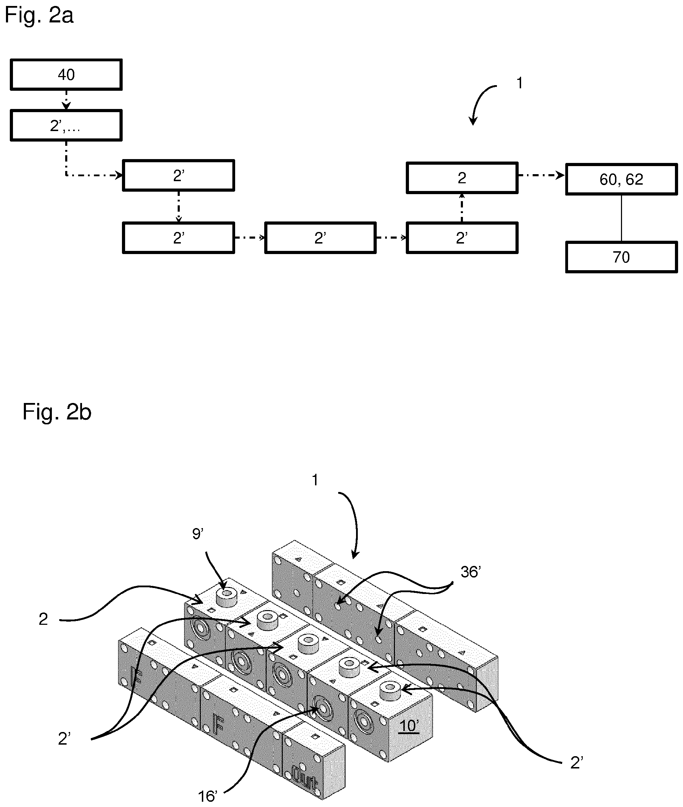

En outre, le temps de résidence du fluide F au sein du réservoir 10 peut encore être avantageusement augmenté en désaxant l'orifice 22 d'injection par rapport à l'orifice 32 de refoulement. Effet, l'orifice 22 d'injection et l'orifice 32 bien qu'étant portés par deux axes et formant donc un plan, ce plan est oblique, c'est-à-dire qu'il n'est ni horizontal ni vertical, comme illustré dans l'exemple de la figure 1b. À cet égard, comme cela est illustré à la figure 1c qui représente une vue rapprochée de la région R encadrée à la figure 2b et comme mentionné précédemment, le premier élément 20 de raccordement comprend un axe longitudinal X1. L'axe longitudinal X1 est un axe central du premier élément 20 de raccordement fluidique passant par l'orifice 22 d'injection. Le deuxième élément 30 de raccordement fluidique, quant à lui, comprend un axe longitudinal X2 agencé de manière similaire que l'axe longitudinal X1 par rapport audit deuxième élément 30 de raccordement fluidique. Les axes longitudinaux X1 et X2 peuvent être sécants ou parallèles. Aux fins de l'augmentation du temps de résidence du fluide F et de l'augmentation des perturbations tourbillonnaires, lesdits axes longitudinaux sont avantageusement séparés d'une distance, d, non nulle. Cette configuration permet également un remplissage « sans bulles » du réservoir 10. En effet, le fluide F entre par l'orifice 22 d'injection, par aspiration, et ressort par l'orifice de refoulement une fois que tout l'air contenu dans ledit réservoir 10 aura été aspiré.

L'orifice 32 de refoulement est ainsi séparé d'une distance, d, non nulle de l'orifice 22 d'injection selon une direction d'axe Y, l'axe Y étant orthogonal aux axes longitudinaux X1 et X2. De préférence, la distance, d, est comprise entre 3 et 4 mm. Cela étant, si lesdits orifices 22, 32 peuvent être séparés d'une distance d dans la direction d'axe Y, il n'est pas exclu qu'ils soient séparés d'une distance d' selon une direction d'axe X dans le même plan et/ou dans un plan XZ, l'axe Z étant orthogonal à l'axe X dans le sens de la section de coupe (sortant du plan de la figure). En cela, la vue en section de la figure 1c peut être trompeuse puisque les première 16 et deuxième 18 ouvertures et respectivement l'orifice 22 d'injection et 32 de refoulement peuvent être situés dans des plans de coupe différents (orthogonaux à l'axe Z).

Si la géométrie de l'écoulement dépend substantiellement des singularités précédemment décrites et donc de la forme des éléments, elle dépend aussi de la vitesse d'écoulement. La forme des éléments du dispositif 2 et la vitesse d'écoulement agissent ainsi en synergie pour obtenir une telle géométrie d'écoulement et permettre une libération continue de plaquettes P à partir des cellules mégacaryocytaires Mk avec un rendement amélioré par rapport aux dispositifs connus. Nous y reviendrons dans la suite.

À cet égard et comme cela a été mentionné précédemment, le système 1 est muni d'un dispositif de pompage 60 (illustré à la figure 1b). Le dispositif de pompage 60 est en communication fluidique avec le réservoir 10 de libération de plaquettes. Il permet de de déplacer le fluide F contenu dans le réservoir 10. Le déplacement du fluide peut être dû à une dépression dans le dispositif 2, mais il peut également être dû à un refoulement du fluide comme cela sera vu plus en détail dans la suite. Ce qui importe ici est que le fluide F puisse circuler avec une vitesse plus importante que celle qu'il présenterait en l'absence du dispositif de pompage 60. Le dispositif de pompage 60 est par exemple une pompe à vide ou de manière générale tout dispositif apte à générer une dépression ou un refoulement de fluide dans le dispositif 2. Par exemple, le dispositif de pompage 60 est une pompe à palette, mais ceci n'est nullement limitatif dans le cadre de la présente invention.

Le dispositif de pompage 60 peut aussi être situé en amont du premier élément 20 de raccordement fluidique dans le sens de l'écoulement, de préférence dans le réservoir 40 source. À cet égard, on peut prévoir un dispositif de pompage 60 par transfert de fluide ou une surpression dans le réservoir 40. Dans cette configuration, le fluide F étant refoulé dans le sens de l'écoulement souhaité, il passe successivement à travers la portion 24 étrécie, l'orifice 22 d'injection, le réservoir 10 et l'orifice 32 de refoulement en adoptant une géométrie d'écoulement résultant de chacune des singularités. Cela dit, l'utilisation d'un dispositif de pompage 60 par transfert de fluide ((pompe à engrenage, péristaltique, etc.) pourrait s'avérer néfaste pour la libération des plaquettes puisqu'il dégraderait probablement les cellules mégacaryocytaires avant même que le processus ait débuté. L'utilisation de ce type de dispositif de pompage 60 n'est pas obligatoire puisqu'un dispositif de type éjecteur pourrait aussi être envisagé. De manière générale, on pourra utiliser tout type de dispositif de pompage 60 à l'exception éventuellement d'une pompe à transfert de fluide.

Le dispositif de pompage 60 peut être en communication fluidique avec le réservoir 10 par l'intermédiaire du deuxième élément 30 de raccordement fluidique. De préférence, il est situé dans le réservoir 50 de réception. En étant ainsi agencé, le dispositif de pompage 60 est donc situé en aval du deuxième élément 30 de raccordement fluidique dans le sens de l'écoulement du fluide F. Cela permet de tirer pleinement avantage des singularités du dispositif 2 et améliore le processus de formation des perturbations tourbillonnaires au sein du réservoir 10, notamment de sa cavité 14.

Revenons à ce propos à la portion 34 de refoulement du deuxième élément 30 de raccordement. Elle peut être avantageusement évasée depuis l'orifice 32 de refoulement mais cela n'est pas essentiel, pour permettre la formation de perturbations tourbillonnaires au sein du réservoir 10. Précisons qu'un tel évasement de la portion 34 de refoulement tiendrait au fait que sa section s'élargit depuis l'orifice 32 de refoulement du fluide en direction du dispositif de pompage 60, i.e. dans le sens de l'écoulement. L'évasement de la portion 34 de refoulement permet au dispositif de pompage 60, lorsque ce dernier est situé en aval du deuxième élément 30 de raccordement, de déplacer plus efficacement le fluide. Ceci s'explique simplement par le fait que le pompage ne serait pas aussi efficace si le deuxième élément 30 de raccordement présentait une section égale à celle de l'orifice 32 de refoulement sur toute sa longueur, sa longueur étant définie comme la distance entre l'orifice 32 de refoulement et une autre extrémité dudit deuxième élément 30. En effet, comme mentionné précédemment, l'orifice 32 de refoulement présente un diamètre d'au plus 1 mm. L'évasement n'a donc d'intérêt sinon celui de permettre une aspiration plus efficace.

Le système 1 comprend en outre un module d'alimentation électrique 62 du dispositif de pompage 60. Le module d'alimentation électrique 62 permet au dispositif de pompage 60 de jouer son rôle moteur et de déplacer le fluide F. De plus, le module d'alimentation électrique 62 permet également de régler la puissance délivrée par le dispositif de pompage 60 et tous autres paramètres utiles que l'homme du métier saura apprécier. D'ailleurs, préférentiellement, le module d'alimentation électrique 62 est configuré de sorte que la puissance délivrée par le dispositif de pompage 60 permette d'ajuster la vitesse de déplacement du fluide F dans le système 1 afin d'obtenir un débit de fluide F compris entre 37 mL/min et 120 mL/min, voire plus. Pour autant, ce n'est pas le seul paramètre influençant le débit de fluide comme nous le verrons dans la suite. Le système 1 peut être d'ailleurs être équipé d'un débitmètre permettant de contrôler le débit de fluide dans le réservoir 10 en fonction de la puissance délivrée par le module d'alimentation électrique 62. À titre d'exemple, un tel débitmètre pourrait être placé entre le réservoir 40 source et le réservoir 10. En outre, le module d'alimentation électrique 62 n'est pas obligatoirement localisé à proximité immédiate du dispositif de pompage 60. Il peut être déporté.

Le système 1 comprend en outre un système de programmation 70 configuré pour contrôler le module d'alimentation électrique 62 du dispositif de pompage 60 afin de mettre en œuvre une ou plusieurs séquences de libération de plaquettes réalisées de sorte à générer un écoulement continu du fluide F entre ledit orifice 22 d'injection et ledit orifice 32 de refoulement ainsi que des perturbations tourbillonnaires au sein du réservoir 10 provoquant la fragmentation des extensions cytoplasmiques Ck des cellules mégacaryocytaires Mk. Le système de programmation 70 comprend au moins un ou plusieurs processeurs aptes à exécuter un programme afin de mettre en œuvre lesdites séquences de libération de plaquettes. Comme cela sera vu plus loin, les séquences de libération de plaquettes peuvent varier dans leurs durées, leur nombre, etc. Cela étant, précisons que si le module d'alimentation électrique 62 peut être contrôlé au moyen d'un tel système de programmation 70 pour une application industrielle, elle peut tout aussi bien être contrôlée manuellement par un opérateur.

En référence aux figures 2a à 2d, dans un deuxième et un troisième mode de réalisation de l'invention, le système 1 comprend, en plus du premier dispositif 2, une pluralité d'autres dispositifs 2' ou 2". Les autres dispositifs 2' et 2" sont identiques au dispositif 2 précédemment décrit. Les traits en pointillés fléchés indiquent le sens de déplacement du fluide F dans le système.

Dans le deuxième mode de réalisation du système 1 selon l'invention illustré aux figures 2a à 2c, le dispositif 2 est connecté en série avec d'autres dispositifs 2'. Les dispositifs 2, 2' sont en communication fluidique les uns à la suite des autres. De préférence, le nombre d'autres dispositifs 2' est compris entre 1 et 4 de sorte que le nombre total de dispositifs 2, 2' est compris entre 1 et 5. Dans une telle configuration, le système 1 permet de manière très avantageuse de multiplier le nombre de singularités et donc d'améliorer encore plus le rendement de libération des plaquettes. En effet, on multiplie ainsi d'autant plus le nombre de turbulences au sein du système 1 qu'il y a d'autres dispositifs 2' de sorte que chaque fois que le fluide F traverse un autre dispositif 2' le rendement de libération de plaquettes est augmenté.

Un exemple de réalisation d'un tel ensemble est illustré à la figure 2b. Il comprend cinq dispositifs 2, 2' montés en série. On distingue les réservoirs 10', les ouvertures 16' (les ouvertures 18' étant opposées et non visibles). Les réservoirs 10' ont une enveloppe extérieure de forme cubique, ce qui ne les empêche pas d'avoir une cavité 14 de toute forme désirée, ici elle est de forme sphérique. Dans l'exemple de réalisation illustré, le réservoir 10' est muni d'une cheminée 9' pour évacuer des bulles d'air. Toutefois ce n'est pas obligatoire. Les éléments de raccordement 20', 30', tout au moins leurs portions 24', 34' et orifices 22', 32' respectifs sont formés dans des pièces d'assemblage, situées de part et d'autre des réservoirs 10'. Les orifices 22', 32' ne sont pas alignés, i.e. ils sont désaxés, les uns par rapport aux autres dans le sens d'écoulement du fluide F, pour chaque autre dispositif 2'.

À ce propos, on peut également préciser que le(s) réservoirs 10, 10', les éléments d'assemblage susmentionnés et les éléments de raccordement 20, 20', 30, 30' peut(vent) être fabriqué(s) par tout procédé de fabrication approprié connu de l'état de la technique. Dans le cas d'espèce, les dispositifs 2, 2' ont été fabriqués par impression 3D et assemblés par vissage. À cet égard, les dispositifs 2, 2' peuvent comprendre des moyens de fixation pour permettre leur assemblage. De préférence, ils sont faits à base de résines de type polyéther imide (PEI) ou de résine photopolymérisable (telles que celles utilisée dans les orthèses dentaires). De préférence, il s'agit d'un matériau répondant à la pharmacopée du pays dans lequel doit être utilisé le système 1 selon l'invention. Le matériau peut ainsi être certifié par l'ANSM (*Agence Nationale de sécurité du médicament et des produits de santé*), l'EMA (*European Medicines Agency*) en France, la PhEU (Pharmacopée Européenne) la PMDA (*Pharmaceutical and medical Devices Agency*) au Japon ou encore la FDA (*Food and Drug administration*) et/ou l'USP (Pharmacopée américaine) aux Etats-Unis, etc.. À titre d'exemple, pour un usage aux Etats-Unis, le dispositif peut être de préférence fait en matériaux biocompatibles classés USP VI, VI désignant la classe USP. Le test de classe USP est l'une des méthodes de test les plus courantes pour déterminer la biocompatibilité des matériaux. Il y a six classes, VI étant la plus rigoureuse. Les tests de classe VI visent à certifier qu'il n'y a pas de réactions nocives ni d'effets physiques à long terme causés par des produits chimiques relargués par des matières plastiques.. Du fait de ces spécificités et du fait que les dispositifs 2' sont clos, ils sont particulièrement bien adaptés pour un traitement du fluide propre à une utilisation médicale.

Une telle configuration est en outre avantageuse en ce qu'elle permet d'améliorer le rendement du système tout en étant agencé de manière optimale. En effet, au lieu de prévoir un dispositif de pompage 60 et une alimentation électrique 62 par dispositif 2, 2', ces éléments sont mutualisés pour deux, trois voire plus de dispositifs 2, 2'. En effet, alors que le dispositif de pompage 60 est en communication fluidique avec le premier dispositif 2 par l'intermédiaire du deuxième élément 30 de raccordement fluidique dudit premier dispositif 2, comme vu précédemment, les autres dispositifs 2' sont reliés directement au premier élément 20, 20' de raccordement du dispositif 2, 2' qui les précèdent. En d'autres termes, seul le premier dispositif 2 est relié au dispositif de pompage 60.

Considérons par exemple le premier autre dispositif 2' directement relié au premier dispositif 2. Le deuxième élément 30' de raccordement fluidique dudit premier autre dispositif 2' est en communication fluidique avec ledit premier élément 20 de raccordement fluidique dudit premier dispositif 2. De préférence, cette communication fluidique est directe. Il est donc en communication fluidique indirecte avec le dispositif de pompage 60 via le premier dispositif 2. Considérons maintenant le deuxième autre dispositif 2' directement relié au premier autre dispositif 2'. Le deuxième élément 30' de raccordement fluidique dudit deuxième autre dispositif 2' est en communication fluidique avec ledit premier élément 20' de raccordement fluidique dudit premier autre dispositif 2'. Ceci vaut également pour les autres dispositifs 2' successifs.

D'autres types d'assemblages, c'est-à-dire de raccordement du dispositif de pompage 60 aux dispositifs 2, 2' peuvent être envisagés pour monter en série les dispositifs 2, 2' tout en respectant le concept inventif de l'invention.

Précisons que, de manière similaire à ce qui a été vu pour le premier mode de réalisation, le système 1 selon ce deuxième mode de réalisation comprend en plus du dispositif de pompage 60, le module d'alimentation électrique 62 du dispositif de pompage 60 et le système de programmation 70 associé. La géométrie de l'écoulement n'est pas modifiée dans un tel système 1. En effet, la vitesse d'écoulement est préservée puisqu'au lieu de générer la dépression ou un refoulement dans le circuit d'un unique dispositif 2, celle-ci se propage dans l'ensemble des dispositifs 2'.

Il peut alors être nécessaire d'adapter la puissance d'aspiration ou de refoulement, selon le dispositif de pompage 60 utilisé, et plus précisément l'augmenter afin qu'un déplacement suffisant du fluide soit obtenu pour le dispositif 2' le plus éloigné. Cependant, une puissance d'aspiration ou de refoulement trop élevée risque de nuire au rendement de libération de plaquettes puisque ce rendement n'est optimal que dans une gamme définie de débit de fluide et donc de puissance d'aspiration ou de refoulement. Il convient donc de réaliser une telle adaptation en fonction de cette contrainte si le dispositif de pompage 60 utilisé ne permet pas de maintenir une pression constante dans le système 1. La plupart des dispositifs de pompage actuellement proposés dans le commerce permettent généralement de s'affranchir de cette problématique.

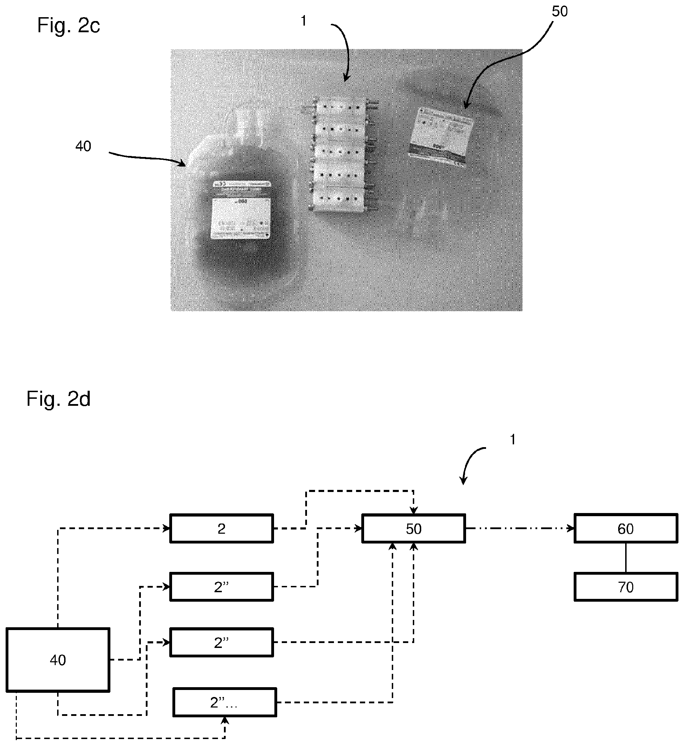

De manière similaire à ce qui a été mentionné précédemment pour le dispositif de pompage 60, le module d'alimentation électrique 62 et le système de programmation 70, il est également possible de ne prévoir qu'un unique réservoir 40 source et un unique réservoir 50 de réception pour l'ensemble du système 1, qu'il comprenne un ou, le cas échéant, une pluralité de dispositifs 2. Une telle réalisation est par exemple illustrée à la figure 2c. Dans cet exemple de réalisation, le réservoir 40 source et le réservoir 50 de réception consistent en des poches souples. Le réservoir 40 source est en communication fluidique avec l'autre dispositif 2' le plus en amont, i.e. le plus éloigné du premier dispositif 2 dans le circuit fluidique. Le réservoir 50 de réception est quant à lui, de manière similaire au dispositif de pompage 60, en communication fluidique avec le premier dispositif 2 par l'intermédiaire du deuxième élément 30 de raccordement fluidique. Rappelons que le dispositif de pompage 60 peut avantageusement être situé dans le réservoir 50 de réception.

Dans un troisième mode de réalisation de l'invention illustré à la figure 2d, le système 1 comprend des dispositifs 2, 2" connectés en parallèle, c'est-à-dire que le premier dispositif 2 est monté en parallèle avec d'autres dispositifs 2". Dans cette configuration, les dispositifs 2, 2" ne sont pas connectés entre eux. Autrement dit, ils sont indépendants les uns par rapport aux autres. Chaque dispositif 2, 2" opère donc indépendamment des autres. Le nombre préférentiel de dispositif(s) 2" n'est pas limité. Plus on augmente le nombre de dispositifs 2" plus la quantité de fluide traitée peut être augmentée. Par exemple, avec un système 1 comprenant trois dispositifs 2, 2" montés en parallèle, il est possible de traiter environ 3 litres de fluide F par heure, ce qui confère à l'invention un avantage considérable par rapport aux dispositifs connus de l'art antérieur. Cette configuration permet d'adapter le nombre de dispositifs 2, 2" en fonctionnement en fonction du volume de fluide F à traiter. On peut prévoir un réservoir 40 source de plus grand volume mais cela n'est pas obligatoire. On pourrait, par exemple, à titre d'alternative prévoir un moyen pour délivrer en continu le fluide à traiter.

Quoiqu'il en soit, de manière similaire à la variante de réalisation de la figure 2b, le dispositif de pompage 60, le module d'alimentation électrique 62 et le système de programmation 70 sont mutualisés pour l'ensemble des dispositifs. Cependant, dans ce cas, le dispositif de pompage 60 peut être, par un jeu de raccords, mis en communication fluidique avec l'ensemble des dispositifs 2, 2". D'autres types d'assemblages peuvent être envisagés pour monter en parallèle les dispositifs 2, 2" tout en respectant le concept inventif de l'invention.

En outre, le système 1 de la présente invention peut être adapté de sorte à intégrer des fonctionnalités supplémentaires, là encore en respectant le concept inventif de l'invention. Par exemple, on pourrait prévoir en entrée du réservoir 10 une tubulure en Y qui permettrait de faire circuler deux types de fluide F et réaliser des mélanges au sein du réservoir 10, qui ont l'a vu, est configuré pour générer des perturbations tourbillonnaires.

L'invention concerne en outre un procédé 5 de libération de plaquettes P à partir d'un fluide F comprenant des cellules mégacaryocytaires Mk comprenant des extensions cytoplasmiques Ck. Le procédé 5 selon l'invention permet, comme nous le verrons de manière détaillée dans les sections qui suivent, de fragmenter des mégacaryocytes, Mk, et/ou des extensions cytoplasmiques, Ck, afin de libérer les plaquettes. À cet égard, le procédé 5 est mis en œuvre au moyen d'un système 1 tel que précédemment décrit.

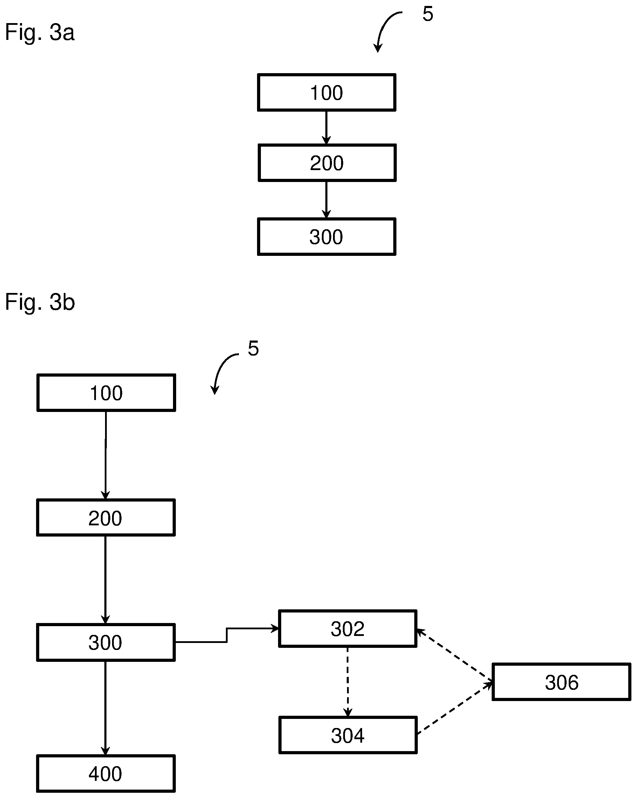

En référence aux figures 3a et 3b, lors d'une première étape 100 du procédé 5, on fournit un fluide F comprenant des mégacaryocytes Mk. De préférence, le fluide F est stocké dans un réservoir 40 source prévu à cet effet. Le fluide F dont il s'agit est par exemple un fluide prélevé sur un patient, notamment au niveau de sa moelle osseuse, ou un fluide obtenu par culture cellulaire. En tout état de cause, ce fluide F comprend des mégacaryocytes Mk et des plaquettes peuvent en être libérées. Précisons néanmoins que quel que soit le fluide F, les mégacaryocytes Mk sont en suspension dans ledit fluide F et que, selon la viscosité du fluide, les mégacaryocytes se répartiront de manière différente.

Compte-tenu des quantités, rappelons-le, industrielles de fluide F qui peuvent être traitées et incidemment de plaquettes qui peuvent être libérées, une étape préalable de culture cellulaire peut être envisagée en vue d'obtenir la quantité de fluide F et de précurseurs (i.e. les mégacaryocytes) souhaitée. Cette étape de culture cellulaire a pour but d'une part de permettre la maturation des mégacaryocytes Mk et d'autre part leur multiplication, i.e. leur prolifération. Le document FR 3 039 166 décrit plus amplement le processus de culture cellulaire.

À titre d'exemple, les mégacaryocytes Mk utilisés dans le fluide F sont des mégacaryocytes dérivés de progéniteurs CD34+ immortalisés ou non. L'étape préalable de culture de tels mégacaryocytes Mk peut comprendre deux phases durant au total entre 11-17 jours. Durant une première phase qui dure entre 5 et 9 jours, les mégacaryocytes Mk sont mis en culture avec un mélange comprenant à titre d'exemple les agents suivants : milieu de culture sans sérum, un cocktail de cytokine contenant de la TPO, IL-6 et de l'IL-9 et l'antagoniste d'AhR et le LDL (Low density lipoprotein destinés à stimuler la prolifération des cellules CD34+ et les engager dans la voie de la mégacaryocytopoïèse, ). Le AhR stimule avantageusement la maturation des mégacaryocytes Mk. Lors d'une seconde phase de cette étape de culture, dont la durée est approximativement de 6-8 jours jours, les cellules obtenues sont mises en culture avec un mélange de SR1 et de thrombopoïétine, TPO, et éventuellement d'autres agents tels qu'énumérés ci-avant en référence à la première phase. Il est à noter que les cellules obtenues comprennent des mégacaryocytes mais pas exclusivement. De plus, il est à noter que toutes les cellules ne donnent pas lieu à des mégacaryocytes Ces phases peuvent durer plus ou moins longtemps selon le nombre de cellules désiré, les agents utilisés, etc..

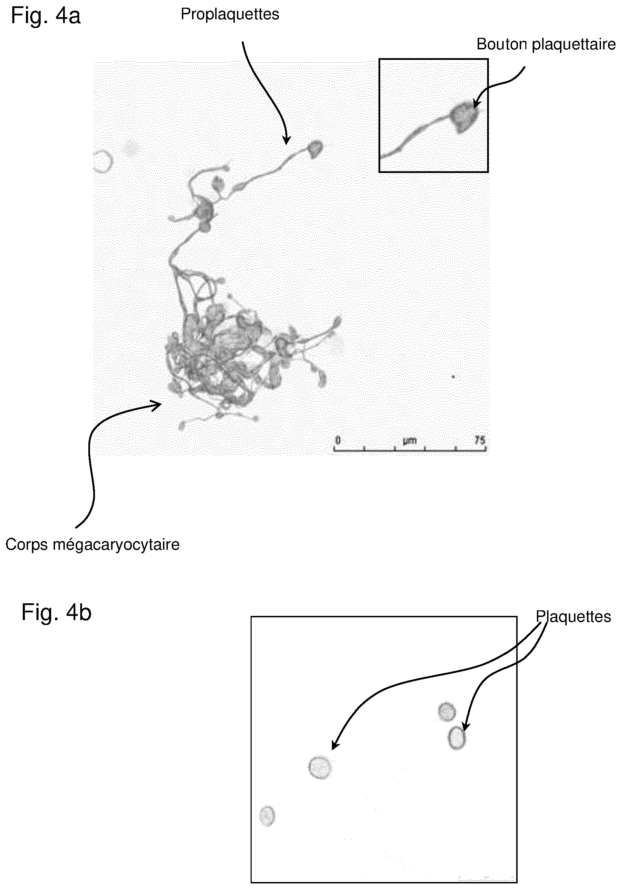

Cette étape préalable ne sera pas décrite plus en détail car elle ne fait pas l'objet de la présente invention et n'en fait partie, l'invention se bornant à proposer un procédé pour la libération des plaquettes à partir du fluide F et non un procédé incluant une quelconque étape de culture. En tout état de cause, à l'issu de cette phase de culture cellulaire on obtient des mégacaryocytes proplaquettaires, c'est-à-dire des mégacaryocytes munies de proplaquettes. Ils sont simplement nommés mégacaryocytes Mk dans la présente invention par souci de simplification. De telles cellules sont illustrées à la figure 4a. Elles comprennent un corps mégacaryocytaire avec des proplaquettes formant des extensions depuis le corps mégacaryocytaire. Les extensions cytoplasmiques sont soutenues par un réseau de microtubules et se terminent par un bouton plaquettaire qui préfigure la future plaquette. Le fluide F fourni à l'étape 100 comprend de telles mégacaryocytes proplaquettaires.

En référence de nouveau aux figures 3a et 3b, le procédé 5 comprend suite à l'étape 100, une étape 200 au cours de laquelle on alimente électriquement le dispositif de pompage 60 de sorte à générer une dépression ou un refoulement en sortie du deuxième élément 30 de raccordement fluidique.

Lors de l'étape 200, on peut définir la puissance de module d'alimentation électrique 62 du dispositif de pompage 60 de sorte à ajuster la puissance d'aspiration ou de refoulement. Plus précisément, il convient d'ajuster la puissance de manière à ce que le débit de fluide F obtenu soit compris entre 37 mL/min et 120 mL/min, voire davantage. Il est à noter que selon le matériau dont est fait le dispositif du système 1 selon l'invention, le débit de fluide peut être significativement augmenté. Il s'agit en effet de la gamme optimale de débit pour obtenir les rendements de libération de plaquettes les plus élevés avec le procédé selon l'invention. La puissance à appliquer dépend intrinsèquement du matériel utilisé pour le dispositif de pompage 60.

Lors d'une étape 300, on commande le système de programmation 70 afin de lancer une ou plusieurs séquences de libération de plaquettes telles que définies précédemment. Le système de programmation 70 permet de contrôler avantageusement le module d'alimentation électrique 62. Il permet aussi de contrôler les durées et le nombre de séquences de libération de plaquettes P. Les séquences de libération de plaquettes peuvent durer aussi longtemps que l'utilisateur le souhaite afin de traiter le volume de fluide souhaité.

Lorsqu'on commande le système de programmation 70 tel que décrit dans la section précédente, le dispositif de pompage 62 étant alimenté, une dépression ou, selon la configuration, un refoulement est généré(e) dans le(s) dispositif(s) 2, 2', 2". On entend par « dépression » le fait que la pression dans le(s) dispositif(s) 2, 2', 2" est inférieure à la pression atmosphérique, c'est-à-dire qu'il(s) soit(ent) soumis à un vide relatif. Le « refoulement » correspond lui au fait de déplacer le fluide F par une pompe refoulante, par exemple de type éjecteur, ou un compresseur. En conséquence de cette dépression ou de ce refoulement, un écoulement continu de fluide F, i.e. un déplacement a lieu dans le système 1 du réservoir 40 source au réservoir 50 de réception. Précisons, qu'à titre préférentiel, la puissance d'aspiration ou de refoulement du dispositif de pompage 60 est maintenue constante pendant le procédé 5 de sorte que la dépression, le cas échéant la vitesse de refoulement, et le débit de fluide F soient également maintenus sensiblement constants. Toutefois, cela n'empêche pas, lors de la mise en œuvre du procédé de varier cette puissance dans des limites permettant d'obtenir le rendement optimal de libération de plaquettes.

Dès que la dépression ou le refoulement est généré(e) dans le(s) système(s) 2, 2', 2", le fluide passe par différentes phases i.e. 302, 304 et 306, qui se produisent de manière continue tant que la dépression ou le refoulement est maintenu(e), sans action supplémentaire sur le système de programmation 70. Ces phases sont la conséquence directe du déplacement de fluide dans le(s) système(s) 2, 2', 2". Une phase 302 correspond à la phase précédant l'entrée du fluide F dans le réservoir 10, une phase 304 correspond à la phase où le fluide F est dans le réservoir 10 et une phase 306 correspond à la phase suivant l'aspiration du fluide F hors du réservoir 10. En d'autres termes, les phases 302, 304 et 306 se produisent donc en pratique simultanément puisque lorsqu'une partie du fluide F est traitée, au même moment une autre partie du fluide F est en train d'être traitée dans le réservoir 10 et une autre partie encore est sur le point l'être en amont du réservoir 10, i.e. dans le premier élément 20 de raccordement ou dans le réservoir 40 source, ceci le long du circuit fluidique.

Lors de la phase 302, le fluide F est aspiré à travers le premier élément 20 de raccordement fluidique. La pression au sein du dispositif étant maintenue constante, la vitesse du fluide F augmente lorsqu'il traverse la portion 24 étrécie, du fait même de l'évasement de ladite portion 24 étrécie. Le fluide F est alors refoulé dans le réservoir 10 via l'orifice 22 d'injection. La singularité tenant à l'élargissement brusque de section présent à l'interface orifice 22 d'injection/réservoir 10 provoque un ralentissement du fluide F lors de son entrée dans le réservoir 10.

Lors de la phase 304, le fluide F circule dans le réservoir 10 en étant soumis à la dépression ou le refoulement généré(e) par le dispositif de pompage 60 dans les limites de la cavité 14. En effet, bien que le fluide ait été ralenti par la singularité existant à l'interface orifice 22/réservoir 10, celui-ci reste en mouvement du fait de cette dépression ou refoulement. Le fluide ne sort pas directement du réservoir ce qui permet la circulation dudit fluide sous la forme de perturbations tourbillonnaires. En effet, le système 1 est configuré de sorte que le temps de résidence du fluide F dans le réservoir 10 est suffisant pour générer des perturbations. Comme nous l'avons vu précédemment, un temps de résidence suffisant est obtenu grâce à la singularité tenant aux dimensions importantes du réservoir 10 par rapport à l'orifice 32 de refoulement. Le temps de résidence peut être également augmenté, mais dans une moindre mesure en désaxant avantageusement l'orifice 22 d'injection par rapport à l'orifice 32 de refoulement sur le trajet du fluide F comme explicité ci-dessous.

Dans ces conditions, des perturbations tourbillonnaires de tailles sensiblement équivalentes à celles des mégacaryocytes sont générées. Elles permettent de fragmenter les mégacaryocytes Mk et leurs extensions cytoplasmiques Ck (proplaquettes), ce qui a pour effet de libérer les plaquettes P. Concomitamment, d'autres produits Dk résultants de la fragmentation des mégacaryocytes sont produits. Ces produits correspondent à des bouts de proplaquettes, des corps de mégacaryocytes MK encore intacts ou encore des morceaux de cytoplasme. Le fluide F étant traité de manière continu dans le temps, ceci pour chaque séquence de libération de plaquettes, les phénomènes décrits ci-dessus sont reproduits continuellement et les plaquettes sont libérées de manière continue. En somme, alors que le fluide F entrant dans le réservoir 10 comprend des cellules mégacaryocytaires Mk comprenant des extensions cytoplasmiques, le fluide F sortant du réservoir 10 comprend essentiellement des plaquettes P et les autres produits, Dk. À ce propos, l'utilisation d'un système 1 comprenant des dispositifs 2, 2' en série, tel qu'illustré aux figures 2a à 2c, permet avantageusement de réduire les proportions de mégacaryocytes encore intacts à la suite du procédé 5. Rappelons qu'à chaque fois que le fluide F circule dans un dispositif 2', il subit des perturbations, d'où les rendements plus élevés d'un système 1 en série en comparaison avec un système 1 comprenant un dispositif 2 unique.

Lors de la phase 306, le fluide F, chargé de plaquettes P et d'autres produits Dk résultant de la fragmentation des mégacaryocytes est aspiré à travers l'orifice 32 de refoulement et plus généralement à travers le deuxième élément 30 de raccordement fluidique. Il atteint ensuite le réservoir 50 de réception, le cas échéant, et le dispositif de pompage 60.

Lors d'une étape 400, la(es) séquence(s) sont arrêtées par arrêt du module d'alimentation électrique 62 au moyen du système de programmation 70.

Ces phases se produisent à l'identique dans tous les autres dispositifs 2' et/ou 2" éventuellement présents dans le système 1 selon l'invention.

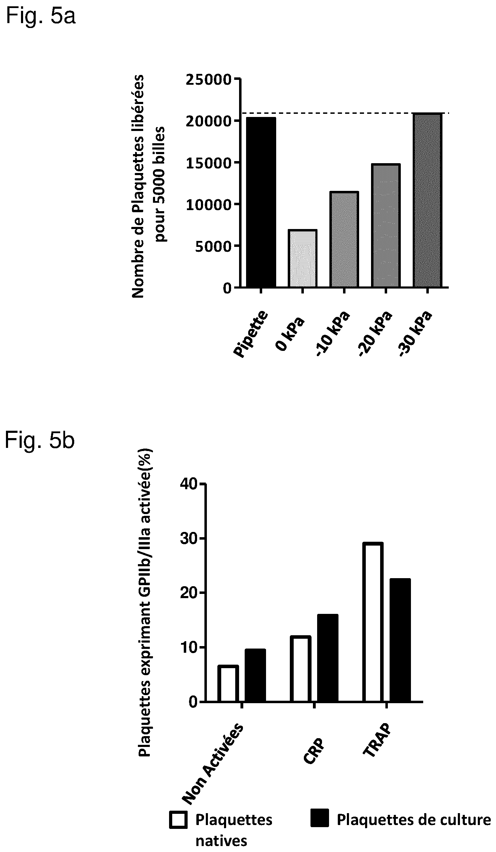

Afin de libérer les plaquettes, le débit de fluide doit être avantageusement compris entre 37 mL/min et 120 mL/min, voire plus. Ces valeurs de débit sont des valeurs moyennes représentant la moyenne de six mesures. La figure 5a illustre l'évolution du nombre de plaquettes pour un nombre de tubes de calibration (BD Trucoun^{®} Tubes) ou de comptage égal à 5000 en fonction du vide relatif généré en mettant en œuvre le procédé selon l'invention (barres grises) et au moyen d'une pipette (barre noire). Le vide relatif est notamment exprimé en kilo Pascal (kPa). Lorsqu'aucune dépression ou refoulement n'est généré(e) dans le(s) dispositif(s) 2, 2', 2", le rendement de libération des plaquettes reste très faible (gris très clair). Avec un vide relatif égal à -10 kPa, on obtient la valeur minimale de 37 mL/min précédemment mentionnée. Avec un tel vide relatif, le rendement de libération des plaquettes augmente et le nombre de plaquettes libérées dépasse 10000 pour 5000 tubes. Ce rendement de libération des plaquettes est encore relativement peu élevé. Plus le vide relatif augmente plus le rendement de libération des plaquettes augmente. Lorsque le vide relatif est égal à -30 kPa, on obtient la valeur de 120 mL/min précédemment mentionnée. Le nombre de plaquettes libérées est supérieur à 20000 pour 5000 tubes de mégacaryocytes. Comme cela est illustré sur la figure 5a, le rendement de libération de plaquettes ainsi obtenu est proche et même supérieur à celui obtenu en utilisant une pipette manuelle classique. Ceci illustre bien l'importance de la dépression ou du refoulement de fluide généré(e) dans le système 1 afin d'obtenir un rendement de libération satisfaisant. Si dans l'exemple de réalisation présenté ici le vide relatif est de -30 kPa, il peut être augmenté au-delà de -30 kPa, c'est-à-dire jusqu'à 50 kPa. Le débit de fluide obtenu est alors nettement supérieur à 120 mL/min.

Ceci s'explique par les caractéristiques du(es) dispositif(s) 2, 2', 2" utilisé(s) dans le présent exemple de réalisation. En effet, pour ajuster le débit, un compromis doit être trouvé entre la puissance d'aspiration ou de refoulement du dispositif de pompage 60, les tailles des orifices 22 d'injection et 32 de refoulement, mais aussi l'état de la surface des éléments du(es) dispositif(s) 2, 2', 2" par lesquels transite effectivement le fluide F, ceci dans les limites de l'invention. Concernant ce dernier aspect, plus l'état de surface (micro rugosité, rugosité, etc.) aura pour effet d'exercer des contraintes sur le fluide F et plus celui-ci sera ralentit et inversement. Afin d'obtenir un rendement de libération de plaquettes encore plus élevé, on pourra par exemple ajuster l'état de surface. Cela peut être effectué en modifiant la matière utilisée pour fabriquer les éléments du(es) dispositifs 2, 2', 2" par lesquels transite le fluide F, en utilisant d'autres procédés de fabrication permettant un contrôle de la structuration à l'échelle micrométrique mais aussi en infusant un composé dans la chambre comme du plasma ou de l'albumine.

La Figure 5b présente une analyse de la fonctionnalité des plaquettes obtenues à une pression de -30 kPa pour des plaquettes natives (barres creuses) et des plaquettes de culture (barres pleines). Avec ce vide relatif, environ 8% des plaquettes natives et 10% des plaquettes de culture non activées expriment la Glycoprotéine GPllb/Illa. Environ 12% des plaquettes natives et 16% des plaquettes de culture activées avec un agoniste du type CRP (*Collagen Related Peptide*) expriment la Glycoprotéine GPIIb/IIIa. Enfin, près de 30% des plaquettes natives et 22% des plaquettes de culture activées avec un agoniste du type TRAP (*Trombine Receptor-Activating Peptides*) expriment la Glycoprotéine GPIIb/IIIa. Les plaquettes obtenues par le procédé selon l'invention sont donc bien activées et par conséquent fonctionnelles.

## Revendications

1. Système (1) pour la libération de plaquettes à partir d'un fluide (F) comprenant notamment des cellules mégacaryocytaires (Mk) comportant des extensions cytoplasmiques (Ck), ledit système (1) comprenant :
- un dispositif (2) comprenant :
∘ un réservoir (10) de libération de plaquettes comprenant une première ouverture (16) et une deuxième ouverture (18),
∘ un premier élément (20) de raccordement fluidique fixé au niveau de ladite première ouverture (16) et adapté pour injecter ledit fluide (F) à l'intérieur dudit réservoir (10), ledit premier élément (20) de raccordement comprenant un orifice (22) d'injection du fluide dans le réservoir (10) de libération de plaquettes et une portion (24) étrécie en direction dudit orifice (22), un élargissement brusque de section existant entre l'orifice (22) d'injection et le réservoir (10),
∘ un deuxième élément (30) de raccordement fluidique fixé au niveau de ladite deuxième ouverture (18), ledit deuxième élément (30) de raccordement comprenant un orifice (32) de refoulement du fluide,
- un dispositif de pompage (60) du fluide (F) en communication fluidique avec le réservoir (10) par l'intermédiaire du deuxième élément (30) de raccordement fluidique ou du premier élément (20) de raccordement fluidique,
- un module d'alimentation électrique (62) du dispositif de pompage (60),
- un système de programmation (70) configuré pour contrôler le module d'alimentation électrique (62) du dispositif de pompage (60), afin de mettre en œuvre une ou plusieurs séquences de libération de plaquettes réalisées de sorte à générer un écoulement continu du fluide (F) entre ledit orifice (22) d'injection et ledit orifice (32) de refoulement ainsi que des perturbations tourbillonnaires au sein du réservoir (10) provoquant la fragmentation des extensions cytoplasmiques (Ck) des cellules mégacaryocytaires (Mk).

2. Système (1) selon la revendication 1, dans lequel la portion (24) étrécie est conique.

3. Système (1) selon la revendication 1, dans lequel le premier élément (20) de raccordement comprend un axe longitudinal (X1) et le deuxième élément (30) de raccordement comprend un axe longitudinal (X2), lesdits axes longitudinaux (X1, X2) étant soit sécants soit parallèles et alors séparés d'une distance, d, non nulle.

4. Système (1) selon l'une quelconque des revendications précédentes, dans lequel l'orifice (22) d'injection présente un diamètre d'ouverture inférieur à 1 mm, un rapport du diamètre d'ouverture de l'orifice (22) d'injection par une largeur de section du réservoir (10) est compris entre 0,02 et 0,1.

5. Système (1) selon la revendication 4, dans lequel le rapport du diamètre d'ouverture de l'orifice (22) d'injection par la largeur de section du réservoir (10) est de 0,05.

6. Système (1) selon l'une quelconque des revendications précédentes, dans lequel l'orifice (32) de refoulement présente un diamètre d'ouverture inférieur à 1 mm, un rapport du diamètre d'ouverture de l'orifice (32) de refoulement par une largeur de section du réservoir (10) est compris entre 0,02 et 0,1.

7. Système (1) selon la revendication 6, dans lequel le rapport du diamètre d'ouverture de l'orifice (32) de refoulement par la largeur de section du réservoir (10) est de 0,05.

8. Système (1) selon l'une quelconque des revendications précédentes, dans lequel le réservoir (10) présente une forme sphérique.

9. Système (1) selon l'une quelconque des revendications précédentes, comprenant :
- un réservoir (40) source pour le stockage du fluide (F), connecté au premier élément (20) de raccordement fluidique pour alimenter le réservoir (10) et
- un réservoir (50) de réception du fluide (F) connecté au deuxième élément (30) de raccordement fluidique pour collecter ledit fluide destiné à être aspiré du réservoir (10).

10. Système (1) selon la revendication 9, dans lequel le dispositif de pompage (60) est situé dans le réservoir (50) de réception.

11. Système (1) selon l'une quelconque des revendications précédentes, dans lequel ledit deuxième élément (30) de raccordement comprend en outre une portion (34) évasée depuis ledit orifice (32) de refoulement du fluide en direction du dispositif de pompage (60).

12. Système (1) selon l'une des revendications précédentes comprenant au moins un autre dispositif (2") comportant:
- un réservoir (10") de libération de plaquettes comprenant une première ouverture (16") et une deuxième ouverture (18"),
- un premier élément (20") de raccordement fluidique fixé au niveau de ladite première ouverture (16") et adapté pour injecter ledit fluide (F) à l'intérieur dudit réservoir (10"), ledit premier élément (20") de raccordement comprenant un orifice (22") d'injection du fluide et une première portion (24") étrécie de sorte à pouvoir accélérer le fluide (F), ladite première portion (24") étrécie débouchant sur ledit orifice (22") d'injection,
- un deuxième élément (30") de raccordement fluidique fixé au niveau de ladite deuxième ouverture (18"), ledit deuxième élément (30") de raccordement comprenant un orifice (32") de refoulement du fluide,
ledit autre dispositif (2") étant agencé parallèlement au premier dispositif (2) et connecté au dispositif de pompage par l'intermédiaire du deuxième élément (30') dudit autre dispositif.

13. Système (1) selon l'une des revendications 1 à 11 comprenant au moins un autre dispositif (2') comportant:
- un réservoir (10') de libération de plaquettes comprenant une première ouverture (16') et une deuxième ouverture (18'),
- un premier élément (20') de raccordement fluidique fixé au niveau de ladite première ouverture (16') et adapté pour injecter ledit fluide (F) à l'intérieur dudit réservoir (10'), ledit premier élément (20') de raccordement comprenant un orifice (22') d'injection du fluide et une première portion (24') étrécie de sorte à pouvoir accélérer le fluide (F), ladite première portion (24') étrécie débouchant sur ledit orifice (22') d'injection,
- un deuxième élément (30') de raccordement fluidique fixé au niveau de ladite deuxième ouverture (18'), ledit deuxième élément (30') de raccordement comprenant un orifice (32') de refoulement du fluide,
ledit autre dispositif étant agencé en série avec le premier dispositif (2), ledit deuxième élément (30') de raccordement dudit autre dispositif (2') étant en communication fluidique avec ledit premier élément (20) de raccordement fluidique dudit premier dispositif (2).

14. Procédé (5) de libération de plaquettes (P) à partir d'un fluide (F) comprenant notamment des cellules mégacaryocytaires (Mk) comprenant des extensions cytoplasmiques (Ck), ledit procédé comprenant les étapes suivantes, mises en œuvre au moyen d'un système (1) selon l'une des revendications 1 à 13:
(100) fournir un fluide (F) comprenant des cellules mégacaryocytaires (Mk) en suspension dans ledit fluide (F), lesdites cellules mégacaryocytaires (Mk) comprenant des extensions cytoplasmiques (Ck),
(200) alimenter électriquement le dispositif de pompage (60),
(300) commander le système de programmation (70) afin de lancer une ou plusieurs séquences de libération de plaquettes,
la ou chaque séquence de libération de plaquettes étant réalisée de sorte à générer un écoulement continu du fluide (F) entre ledit orifice (22) d'injection et ledit orifice (32) de refoulement ainsi que des perturbations tourbillonnaires au sein du réservoir (10) provoquant la fragmentation des extensions cytoplasmiques (Ck) des cellules mégacaryocytaires (Mk).

15. Procédé (5) selon la revendication 14, dans lequel, lors de l'étape (200), un vide relatif compris entre -10 kPa et -50 kPa est généré dans le dispositif (2, 2', 2").

## Patentansprüche

1. System (1) für die Freisetzung von Plättchen ausgehend von einem Fluid (F), das insbesondere Megakaryozytenzellen (Mk) umfasst, die zytoplasmatische Erweiterungen (Ck) umfassen, wobei das System (1) umfasst:
- eine Vorrichtung (2), umfassend:
- einen Behälter (10) zur Freisetzung von Plättchen, umfassend eine erste Öffnung (16) und eine zweite Öffnung (18),
- ein erstes Element (20) zur fluidischen Verbindung, das auf Höhe der ersten Öffnung (16) befestigt und angepasst ist zum Injizieren des Fluid (F) ins Innere des Behälters (10), wobei das erste Element (20) zur Verbindung eine Bohrung (22) zur Injektion des Fluids in den Behälter (10) zur Freisetzung von Plättchen und einen verengten Abschnitt (24) in Richtung der Bohrung (22) umfasst, wobei zwischen der Bohrung (22) zur Injektion und dem Behälter (10) eine abrupte Ausweitung des Querschnitts besteht,
- ein zweites Element (30) zur fluidischen Verbindung, das auf Höhe der zweiten Öffnung (18) befestigt ist, wobei das zweite Element (30) zur Verbindung eine Bohrung (32) zur Rückströmung von Fluid umfasst,
- eine Pumpvorrichtung (60) des Fluids (F), die über das zweite Element (30) zur fluidischen Verbindung oder das erste Element (20) zur fluidischen Verbindung in strömungstechnischer Kommunikation mit dem Behälter (10) steht,
- ein Stromversorgungsmodul (62) der Pumpvorrichtung (60),
- ein Programmierungssystem (70), konfiguriert zum Steuern des Stromversorgungsmoduls (62) der Pumpvorrichtung (60), um eine oder mehrere Sequenzen zur Freisetzung von Plättchen derart durchzuführen, um einen kontinuierlichen Durchfluss des Fluids (F) zwischen der Bohrung (22) zur Injektion und der Bohrung (32) zur Rückströmung sowie turbulente Perturbationen innerhalb des Behälters (10) zu erzeugen, wodurch die Fragmentierung der zytoplasmatischen Erweiterungen (Ck) der Megakaryozytenzellen (Mk) hervorgerufen wird.

2. System (1) nach Anspruch 1, wobei der verengte Abschnitt (24) konisch ist.

3. System (1) nach Anspruch 1, wobei das erste Element (20) zur Verbindung eine Längsachse (X1) umfasst und das zweite Element (30) zur Verbindung eine Längsachse (X2) umfasst, wobei die Längsachsen (X1, X2) sich entweder schneiden oder parallel sind und somit um eine Distanz, d, ungleich Null getrennt sind.

4. System (1) nach einem der vorstehenden Ansprüche, wobei die Bohrung (22) zur Injektion einen Öffnungsdurchmesser von weniger als 1 mm aufweist, wobei ein Verhältnis des Öffnungsdurchmessers der Bohrung (22) zur Injektion zu einer Größe des Querschnitts des Behälters (10) zwischen 0,02 und 0,1 liegt.

5. System (1) nach Anspruch 4, wobei das Verhältnis des Öffnungsdurchmessers der Bohrung (22) zur Injektion zu der Größe des Querschnitts des Behälters (10) 0,05 beträgt.

6. System (1) nach einem der vorstehenden Ansprüche, wobei die Bohrung (32) zur Rückströmung einen Öffnungsdurchmesser von weniger als 1 mm aufweist, wobei ein Verhältnis des Öffnungsdurchmessers der Bohrung (32) zur Rückströmung zu einer Größe des Querschnitts des Behälters (10) zwischen 0,02 und 0,1 liegt.

7. System (1) nach Anspruch 6, wobei das Verhältnis des Öffnungsdurchmessers der Bohrung (32) zur Rückströmung zu der Größe des Querschnitts des Behälters (10) 0,05 beträgt.

8. System (1) nach einem der vorstehenden Ansprüche, wobei der Behälter (10) eine Kugelform aufweist.

9. System (1) nach einem der vorstehenden Ansprüche, umfassend:
- einen Quellenbehälter (40) zur Aufbewahrung des Fluids (F), der mit dem ersten Element (20) zur fluidischen Verbindung zum Versorgen des Behälters (10) verbunden ist, und
- einen Aufnahmebehälter (50) des Fluids (F), der mit dem zweiten Element (30) zur fluidischen Verbindung zum Sammeln des Fluids verbunden ist, das dazu bestimmt ist, aus dem Behälter (10) abgesaugt zu werden.

10. System (1) nach Anspruch 9, wobei die Pumpvorrichtung (60) sich in dem Aufnahmebehälter (50) befindet.

11. System (1) nach einem der vorstehenden Ansprüche, wobei das zweite Element (30) zur Verbindung weiter ab der Bohrung (32) zur Rückströmung des Fluids in Richtung der Pumpvorrichtung (60) einen aufgeweiteten Abschnitt (34) umfasst.

12. System (1) nach einem der vorstehenden Ansprüche, umfassend mindestens eine andere Vorrichtung (2"), umfassend:
- einen Behälter (10") zur Freisetzung von Plättchen, umfassend eine erste Öffnung (16") und eine zweite Öffnung (18"),
- ein erstes Element (20") zur fluidischen Verbindung, das auf Höhe der ersten Öffnung (16") befestigt und angepasst ist zum Injizieren des Fluids (F) ins Innere des Behälters (10"), wobei das erste Element (20") zur Verbindung eine Bohrung (22") zur Injektion des Fluids und einen ersten verengten Abschnitt (24") derart umfasst, um das Fluid (F) beschleunigen zu können, wobei der erste verengte Abschnitt (24") an der Bohrung (22") zur Injektion mündet,
- ein zweites Element (30") zur fluidischen Verbindung, das auf Höhe der zweiten Öffnung (18") befestigt ist, wobei das zweite Element (30") zur Verbindung eine Bohrung (32") zur Rückströmung des Fluids umfasst,
wobei die andere Vorrichtung (2") parallel zur ersten Vorrichtung (2) angeordnet und über das zweite Element (30') der anderen Vorrichtung mit der Pumpvorrichtung verbunden ist.

13. System (1) nach einem der Ansprüche 1 bis 11, umfassend mindestens eine andere Vorrichtung (2'), umfassend:
- einen Behälter (10') zur Freisetzung von Plättchen, umfassend eine erste Öffnung (16') und eine zweite Öffnung (18'),
- ein erstes Element (20') zur fluidischen Verbindung, das auf Höhe der ersten Öffnung (16') befestigt und angepasst ist zum Injizieren des Fluids (F) ins Innere des Behälters (10'), wobei das erste Element (20') zur Verbindung eine Bohrung (22') zur Injektion des Fluids und einen ersten verengten Abschnitt (24') derart umfasst, um das Fluid (F) beschleunigen zu können, wobei der erste verengte Abschnitt (24') an der Bohrung (22') zur Injektion mündet,
- ein zweites Element (30') zur fluidischen Verbindung, das auf Höhe der zweiten Öffnung (18') befestigt ist, wobei das zweite Element (30') zur Verbindung eine Bohrung (32') zur Rückströmung des Fluids umfasst,
wobei die andere Vorrichtung mit der ersten Vorrichtung (2) in Reihe angeordnet ist, wobei das zweite Element (30') zur Verbindung der anderen Vorrichtung (2') in strömungstechnischer Kommunikation mit dem ersten Element (20) zur fluidischen Verbindung der ersten Vorrichtung (2) steht.

14. Verfahren (5) zur Freisetzung von Plättchen (P) ausgehend von einem Fluid (F), das insbesondere Megakaryozytenzellen (Mk) umfasst, die zytoplasmatische Erweiterungen (Ck) umfassen, wobei das Verfahren die folgenden Schritte umfasst, durchgeführt mittels eines Systems (1) nach einem der Ansprüche 1 bis 13:
(100) Bereitstellen eines Fluids (F), das Megakaryozytenzellen (Mk) in Suspension in dem Fluid (F) umfasst, wobei die Megakaryozytenzellen (Mk) zytoplasmatische Erweiterungen (Ck) umfassen,
(200) elektrisches Versorgen der Pumpvorrichtung (60),
(300) Steuern des Programmierungssystems (70), um eine oder mehrere Sequenzen zur Freisetzung von Plättchen zu starten,
wobei die oder jede Sequenz zur Freisetzung von Plättchen derart durchgeführt wird, um einen kontinuierlichen Durchfluss des Fluids (F) zwischen der Bohrung (22) zur Injektion und der Bohrung (32) zur Rückströmung sowie turbulente Perturbationen innerhalb des Behälters (10) zu erzeugen, wodurch die Fragmentierung der zytoplasmatischen Erweiterungen (Ck) der Megakaryozytenzellen (Mk) hervorgerufen wird.

15. Verfahren (5) nach Anspruch 14, wobei, bei Schritt (200), ein relatives Vakuum zwischen -10 kPa und -50 kPa in der Vorrichtung (2, 2', 2") erzeugt wird.

## Claims

1. A system (1) for releasing platelets from a fluid (F) comprising in particular megakaryocytic cells (Mk) comprising cytoplasmic extensions (Ck), said system (1) comprising:
- a device (2) comprising:
∘ a platelet release reservoir (10) comprising a first opening (16) and a second opening (18),
∘ a first fluidic connecting element (20) attached at the level of said first opening (16) and adapted to inject said fluid (F) into said reservoir (10), said first connecting element (20) comprising an orifice (22) for injecting the fluid into the platelet release reservoir (10) and a portion (24) narrowing towards said orifice (22), there being an abrupt widening of cross-section between the injection orifice (22) and the reservoir (10),
∘ a second fluidic connecting element (30) attached at the level of said second opening (18), said second connecting element (30) comprising an orifice (32) for discharging the fluid,
- a device (60) for pumping the fluid (F) in fluidic communication with the reservoir (10) by means of the second fluidic connecting element (30) or the first fluidic connecting element (20),
- a power supply module (62) for the pumping device (60),
- a programming system (70) configured to control the power supply module (62) for the pumping device (60), to implement one or more platelet release sequences designed to generate a continuous flow of the fluid (F) between said injection orifice (22) and said discharge orifice (32) and vortex disturbances within the reservoir (10) causing the fragmentation of the cytoplasmic extensions (Ck) of the megakaryocytic cells (Mk).

2. The system (1) according to claim 1, wherein the narrowing portion (24) is conical.

3. The system (1) according to claim 1, wherein the first connecting element (20) comprises a longitudinal axis (X1) and the second connecting element (30) comprises a longitudinal axis (X2), said longitudinal axes (X1, X2) being either intersecting or parallel and then separated by a non-zero distance, d.

4. The system (1) according to any one of the preceding claims, wherein the injection orifice (22) has an opening diameter of less than 1 mm, a ratio of the opening diameter of the injection orifice (22) by a sectional width of the reservoir (10) is between 0.02 and 0.1.

5. The system (1) according to claim 4, wherein the ratio of the opening diameter of the injection orifice (22) to the cross-sectional width of the reservoir (10) is 0.05.

6. The system (1) according to any one of the preceding claims, wherein the discharge orifice (32) has an opening diameter of less than 1 mm, a ratio of the opening diameter of the discharge orifice (32) to a sectional width of the reservoir (10) is between 0.02 and 0.1.

7. The system (1) according to claim 6, wherein the ratio of the opening diameter of the discharge orifice (32) to the cross-sectional width of the reservoir (10) is 0.05.

8. The system (1) according to any one of the preceding claims, wherein the reservoir (10) has a spherical shape.

9. The system (1) according to any of the preceding claims, comprising:
- a source reservoir (40) for the storage of the fluid (F), connected to the first fluidic connecting element (20) for supplying the reservoir (10) and
- a reservoir (50) for receiving the fluid (F) connected to the second fluidic connecting element (30) to collect said fluid intended to be sucked from the reservoir (10).

10. The system (1) according to claim 9, wherein the pumping device (60) is located in the receiving reservoir (50).

11. The system (1) according to any one of the preceding claims, wherein said second connecting element (30) further comprises a portion (34) flared from said orifice (32) for discharging the fluid towards the pumping device (60).

12. The system (1) according to one of the preceding claims comprising at least one other device (2") comprising:
- a platelet release reservoir (10") comprising a first opening (16") and a second opening (18"),
- a first fluidic connecting element (20") attached at the level of said first opening (16") and adapted to inject said fluid (F) inside said reservoir (10"), said first connecting element (20") comprising an orifice (22") for injecting the fluid and a first narrowing portion (24") so as to be able to accelerate the fluid (F), said first narrowing portion (24") opening onto said injection orifice (22"),
- a second fluidic connecting element (30") attached at the level of said second opening (18"), said second connecting element (30") comprising an orifice (32") for discharging the fluid,
said other device (2") being arranged parallel to the first device (2) and connected to the pumping device by means of the second element (30') of said other device.

13. The system (1) according to any of claims 1 to 11 comprising at least one other device (2') comprising:
- a platelet release reservoir (10') comprising a first opening (16') and a second opening (18'),
- a first fluidic connecting element (20') attached at the level of said first opening (16') and adapted to inject said fluid (F) inside said reservoir (10'), said first connecting element (20') comprising an orifice (22') for injecting the fluid and a first narrowing portion (24') so as to be able to accelerate the fluid (F), said first narrowing portion (24') opening out on said injection orifice (22'),
- a second fluidic connecting element (30') attached at the level of said second opening (18'), said second connecting element (30') comprising an orifice (32') for discharging the fluid,
said other device being arranged in series with the first device (2), said second connecting element (30') of said other device (2') being in fluidic communication with said first fluidic connecting element (20) of said first device (2).

14. A method (5) for releasing platelets (P) from a fluid (F) comprising in particular megakaryocytic cells (Mk) comprising cytoplasmic extensions (Ck), said method comprising the following steps, implemented by means of a system (1) according to one of the claims 1 to 13:
(100) providing a fluid (F) comprising megakaryocytic cells (Mk) suspended in said fluid (F), said megakaryocytic cells (Mk) comprising cytoplasmic extensions (Ck),
(200) electrically powering the pumping device (60),
(300) controlling the programming system (70) to initiate one or more platelet release sequences,
the or each platelet release sequence being carried out so as to generate a continuous flow of the fluid (F) between said injection orifice (22) and said discharge orifice (32) and vortex disturbances within the reservoir (10) causing the fragmentation of the cytoplasmic extensions (Ck) of the megakaryocytic cells (Mk).

15. The method (5) according to claim 14, wherein during the step (200) a relative vacuum of between -10 kPa and -50 kPa is generated in the device (2, 2', 2").
